# FASCICULE DE BREVET EUROPEEN

(11) **EP 0 687 462 B1**
(45) Date de publication et mention de la délivrance du brevet: **16.10.1996**
(21) Numéro de dépôt: 95401086.4
(22) Date de dépôt: 10.05.1995
(51) Int. Cl.: A61K 7/48, A61K 7/06, A61K 7/42, C08F 283/00, C08L 51/08

(54) **Composition cosmétique ou pharmaceutique comprenant une dispersion aqueuse de polymère**
Kosmetische oder pharmazeutische Zusammensetzung enthaltende eine wässrige Polymerdispersion
Cosmetic or pharmaceutical composition comprising an aqueous polymer dispersion

(30) Priorité: 08.06.1994 FR 9407017
(43) Date de publication de la demande: 20.12.1995
(73) Titulaire: L'OREAL, F-75008 Paris (FR)
(72) Inventeur: Lion, Bertrand, F-93190 Livry Gargan (FR); Mondet, Jean, F-93700 Drancy (FR); Cazeneuve, Colette, F-75015 Paris (FR)
(74) Mandataire: Dodin, Catherine

(56) Documents cités:
- EP-A- 0 297 576
- EP-A- 0 309 114
- FR-A- 2 680 684
- US-A- 4 198 330

## Description

La présente invention a trait à une composition cosmétique ou pharmaceutique comprenant une dispersion aqueuse de polymères, ainsi qu'à l'utilisation de ladite dispersion en tant qu'agent filmogène dans une composition cosmétique ou pharmaceutique.

Il est connu d'utiliser des dispersions aqueuses de polymères dans des compositions cosmétiques ou pharmaceutiques. Ces dispersions sont généralement utilisées en tant qu'agent filmogène, en particulier pour les produits de maquillage tels que les vernis à ongles et les produits capillaires.
Il est connu, par exemple par la demande de brevet EP 418 469, d'employer dans des vernis à ongles, une dispersion aqueuse de polyuréthanne, seule ou en combinaison avec une dispersion aqueuse d'esters vinyliques et/ou acryliques. Les polyuréthannes en dispersion aqueuse sont également décrits dans les demandes EP 391 322 et EP 214 626, soit pour une application en vernis à ongles, soit pour une application capillaire.
Les propriétés des dispersions aqueuses ainsi obtenues dépendent de la nature des polymères, et donc des monomères, à partir desquels elles sont préparées. Il peut toutefois être intéressant de pouvoir modifier légèrement ces propriétés, par exemple en accentuant/optimisant une propriété particulièrement intéressante, ou en en développant une nouvelle que ladite dispersion ne pourrait avoir de par la nature du polymère qu'elle comprend.

L'invention a pour but de proposer une composition comprenant une dispersion aqueuse d'un polymère choisi dans le groupe constitué par les polyuréthannes et les polyurées, présentant des propriétés améliorées par rapport aux dispersions de polyuréthanne et/ou de polyurée de l'art antérieur.

Un objet de l'invention est donc une composition cosmétique ou pharmaceutique comprenant une dispersion aqueuse de polymère constituée par des particules résultant de la polymérisation radicalaire d'au moins un monomère radicalaire, à l'intérieur et/ou partiellement en surface, de particules préexistantes d'au moins un polymère choisi dans le groupe constitué par les polyuréthannes et/ou les polyurées.
Un autre objet de l'invention est l'utilisation d'une telle dispersion dans une composition cosmétique ou pharmaceutique, en tant qu'agent filmogène.

Dans la suite de la présente description, on entend par "polyuréthanne", tout polymère, seul ou en mélange, choisi dans le groupe constitué par les polyuréthannes et les polyurées.

On a constaté que les compositions selon l'invention, comprenant des dispersions aqueuses de polymères hybrides de polyuréthannes, présentent des propriétés particulières, propriétés qu'il n'est pas possible d'obtenir en utilisant, par exemple, un simple mélange de dispersions aqueuses préexistantes de polyuréthanne et de polymères acrylique et/ou vinylique.
Un autre avantage de la présente invention est de pouvoir, à partir d'une dispersion aqueuse de polymère déjà existante, développer et/ou optimiser certaines propriétés particulièrement intéressantes, de manière relativement contrôlée.

Afin de préparer la composition selon l'invention, on prépare tout d'abord une dispersion aqueuse de polyuréthanne.
Cette dispersion peut être préparée par l'homme du métier sur base de ses connaissances techniques générales, en particulier de la manière suivante.
On dissout le polymère de polyuréthanne, insoluble dans l'eau, dans un solvant organique faiblement soluble dans l'eau, on ajoute de l'eau à cette solution et l'on mélange de manière à former une émulsion, puis l'on évapore le solvant organique de manière à obtenir une dispersion aqueuse du polymère de polyuréthanne dans l'eau présentant un taux de matière sèche d'environ 30-50% en poids.

La dispersion aqueuse de "polyuréthanne" utilisée peut être une dispersion aqueuse de polyuréthanne anionique, cationique ou amphotère, de polyester-polyuréthanne, de polyéther-polyuréthanne et/ou de polyurée, seul ou en mélange.
Ledit polyuréthanne peut être, par exemple, un copolymère polyuréthanne, polyurée/uréthanne ou polyurée, aliphatique, cycloaliphatique ou aromatique, comportant, seule ou en mélange,
. au moins une séquence d'origine polyester aliphatique linéaire ou ramifié et/ou cycloaliphatique et/ou aromatique, et/ou
. au moins une séquence d'origine polyéther aliphatique et/ou cycloaliphatique et/ou aromatique, et/ou
. au moins une séquence siliconée, substituée ou non, ramifiée ou non, par exemple polydiméthylsiloxane ou polyméthylphénylsiloxane, et/ou
. au moins une séquence comportant des groupes fluorés.

Les polyuréthannes tels que définis dans l'invention peuvent être également obtenus à partir de polyesters, ramifiés ou non, ou d'alkydes comportant des hydrogènes mobiles que l'on modifie par réaction avec un diisocyanate et un composé organique bifonctionnel (par exemple dihydro, diamino ou hydroxyamino), comportant en plus soit un groupement acide carboxylique ou carboxylate, soit un groupement acide sulfonique ou sulfonate, soit encore un groupement amine tertiaire neutralisable ou un groupement ammonium quaternaire.

La dispersion aqueuse de polymères hybrides de polyuréthanne selon l'invention est obtenue par polymérisation radicalaire d'au moins un monomère à l'intérieur et/ou partiellement en surface de particules préexistantes de polyuréthanne.

Le monomère radicalaire peut être de nature vinylique ou acrylique, et peut être anionique, cationique, non ionique ou amphotère. On peut également utiliser un mélange de monomères de nature différente. Le monomère, ou le mélange de monomères, est de préférence insoluble ou faiblement soluble dans l'eau.
Parmi les monomères susceptibles d'être employés, on peut citer les esters d'acide acrylique ou méthacrylique, tels que les acrylate ou méthacrylate de méthyle, d'éthyle, de propyle, de butyle, d'isobutyle, de tertiobutyle, et d'éthyl-2-hexyle; les acrylamides ou méthacrylamides N-substituées ou N,N-substituées; les esters vinyliques tels que l'acétate de vinyle; le styrène.
On peut également utiliser, seul ou en mélange, un monomère vinylique, acrylique ou méthacrylique comportant un ou plusieurs groupes siloxanes, en particulier
. le monomère de formule

   CH₂=C(CH₃)-C(O)-O-(CH₂)₃-Si-[O-Si(CH₃)₃]₃
. un macromonomère siliconé à terminaison monofonctionnelle vinylique, allylique, ester éther ou amide de l'acide acrylique ou méthacrylique, de formule

   CH₂=C(R1)-C(O)-X-(CH₂)ₚ -[Si(CH₃)(R4)-O-]ₙ-Si(CH₃)₂-R3

   dans laquelle R1 représente H ou CH₃, X représente O ou NH, p est un entier pouvant être nul, R3 et R4 représentent de manière indépendante CH₃ ou un groupe aliphatique, cycloaliphatique ou aromatique, et n est un entier.

On peut également utiliser un monomère vinylique, allylique, ester éther ou amide de l'acide acrylique ou métacrylique comportant un ou plusieurs groupes halogénés, en particulier chlorés et/ou fluorés, et/ou comportant un groupe absorbant dans l'UVA et/ou l'UVB et pouvant apporter une protection solaire après polymérisation, en particulier les groupements benzylidène camphre et benzotriazole, substitués ou non, parmi lesquels on peut citer le 2-(2'-hydroxy-5-methacrylyl-oxyéthylphényl)-2-H-benzotriazole.

Lorsque le monomère, ou le mélange de monomères, est sous forme liquide à température ambiante, la polymérisation radicalaire peut être effectuée sans employer de solvant.
Lorsque le monomère, ou le mélange de monomères, est sous forme solide à température ambiante, on peut le dissoudre avant la polymérisation, de préférence dans un solvant organique, par exemple polaire et miscible à l'eau, tel que le méthanol. Dans ce cas, après polymérisation, on peut distiller, si cela est nécessaire, le solvant organique contenu dans la dispersion de polymères.

La préparation des dispersions aqueuse selon l'invention est faite dans des conditions telles que le monomère polymérise à l'intérieur et/ou partiellement en surface des particules du polymère en l'absence de toute nucléation, c'est-à-dire sans qu'il n'y ait de formation de particules nouvelles.
Pour ce faire, on peut introduire le polymère de polyuréthanne en dispersion aqueuse présentant un taux de matière sèche de 30-50% en poids dans un réacteur de polymérisation. On peut alors y ajouter le monomère, ou le mélange de monomères, tel quel ou en solution dans un solvant adéquat, ainsi qu'un amorceur de polymérisation radicalaire.
Selon sa nature, on introduit l'amorceur radicalaire soit sous forme de solution dans un solvant organique, soit sous forme de solution aqueuse, soit encore dissout dans le mélange de monomères.
Dans le premier cas, il peut être ajouté en même temps que le monomère en solution, et dans le second cas, il peut être ajouté après le monomère.
On peut utiliser un amorceur de polymérisation radicalaire organique, non soluble dans l'eau, de type peroxyde ou percarbonate, tel que le tertiobutylperoxy-2-éthylhexanoate, ou un amorceur organique soluble dans l'eau, ou encore un amorceur minéral tel que le persulfate de potassium.

On prépare donc le mélange comprenant le polymère de polyuréthanne, le monomère ou le mélange de monomères et l'amorceur de polymérisation.
On chauffe alors ce mélange jusqu'à la température nécessaire de manière à permettre la décomposition de l'amorceur, et l'on poursuit la polymérisation jusqu'à épuisement des monomères.
Il est à noter que la polymérisation peut donc être effectuée en l'absence d'un tensioactif dans la dispersion de polyuréthanne.
On obtient ainsi une dispersion aqueuse de polymère hybride de polyuréthanne.
Les particules constituant cette dispersion finale se présentent sous forme de particules composites, semblables à un "alliage" des deux polymères de base et de taille comparable à celles des particules de polyuréthanne avant polymérisation radicalaire.
Les dispersions ainsi obtenues possèdent des propriétés qui leur sont propres, différentes de celles que l'on obtiendrait en mélangeant deux dispersions aqueuses de chacun des constituants.

Les dispersions qu'on utilise comme agent filmogène dans des compositions cosmétiques ou pharmaceutiques selon l'invention peuvent être mises en oeuvre, de la même manière qu'une dispersion aqueuse de polymère selon l'état de la technique.
On peut par exemple utiliser les dispersions comme agent filmogène dans des produits capillaires tels que des laques ou des shampooings, des lotions ou des mousses de coiffage, dans des produits de maquillage tels que des vernis à ongles ou des mascaras, ou encore dans des bases de soin pour ongles ou des produits pour le soin de la peau.
On peut également utiliser les dispersions selon l'invention dans des produits destinés à la photoprotection de la peau et/ou des cheveux contre le rayonnement ultraviolet, en particulier contre le rayonnement solaire, lorsqu'elles contiennent un monomère adéquat, susceptible d'apporter une certaine protection solaire.

L'invention est illustrée plus en détail dans les exemples suivants, dans lesquels les pourcentages sont donnés en poids. La mesure de dureté est effectuée au pendue de Persoz, à une température de 30°C et à une humidité relative (HR) de 50%.

### Exemple 1

On introduit dans un réacteur préchauffé à environ 90°C, 195,5 g de dispersion aqueuse de polyester-polyuréthanne à groupes carboxyliques (taux de matière sèche : 35,8 %) vendue sous le nom de SANCUR 815 par Sancor.
On ajoute 224,6 ml d'eau permutée et on laisse sous agitation et barbotage d'azote, à environ 80°C, pendant 15 minutes.
On ajoute alors 30 g de méthacrylate de méthyle au goutte à goutte, ce qui prend environ 45 minutes, puis on laisse sous agitation pendant 1 heure à 80°C.
On ajoute 0,5 ml de tertbutylperoxy-2-éthylhexanoate (Trigonox 21S de Akzo) et on laisse réagir 6 heures sous agitation et barbotage d'azote à 80°C.
Le mélange alors obtenu à la même apparence qu'au départ bien que tout le monomère ait polymérisé.
On descend la température du mélange réactionnel à 25°C et l'on concentre la dispersion sous pression réduite jusqu'à obtention d'un taux de matière sèche de 40%.
On obtient ainsi une dispersion qui, après filtration sur toile de Nylon, a les caractéristiques suivantes:
. taille moyenne des particules déterminée par un appareil de diffusion quasi-élastique de lumière du type Coulter N4, de Coultronix: 36 nm
. polydispersité: 0,2
   Sachant que la taille des particules dans la dispersion initiale de polyuréthanne (SANCUR 815) est de 31 nm avec une polydispersité de 0,25, on constate que la polymérisation du monomère n'a presque pas modifié la taille des particules initiales.
. absence de double distribution de particules, ce qui signifie que, lors de la polymérisation, on n'a pas créé une seconde population de particules, en plus de la population initiale.
   La dispersion obtenue est une dispersion aqueuse d'un polymère hybride dont les particules résultent de la polymérisation radicalaire d'un monomère méthacrylate de méthyle, à l'intérieur et/ou partiellement en surface, des particules d'un polymère préexistant de polyuréthanne.

### Exemples 2 à 10

De manière similaire à celle décrite dans l'exemple 1, on prépare à partir d'une dispersion aqueuse de polyester-polyuréthanne à groupes carboxyliques (taux de matière sèche : 35,8 %) vendue sous le nom de SANCUR 815 par Sancor, différents polymères hybrides selon le tableau ci-dessous.
L'amorceur de polymérisation est Trigonox 21S (0,5 ml).

Les mesures de taille de particules et de polydispersité sont effectuées pour une dispersion ayant un taux de matière sèche de 40%.

| | dispersion de polyuréthanne | eau ajoutée | monomère | taille des particules | polydispersité |
|---|---|---|---|---|---|
| exemple 2 | 167,6 g | 192,4 g | 40 g de méthacrylate de méthyle | 45 nm | 0,18 |
| exemple 3 | 209,5 g | 240,7 g | 25 g de méthacrylate de méthyle | 33 nm | 0,10 |
| exemple 4 | 209,5 g | 240,7 g | 25 g de méthacrylate de cyclohexyle | 36 nm | 0,15 |
| exemple 5 | 209,5 g | 240,7 g | 25 g d'acrylate d'isobornyle | 30 nm | 0,17 |
| exemple 6 | 223,4 g | 256,7 g | 20 g d'acrylate d'éthyl-2-hexyle | 35 nm | 0,17 |
| exemple 7 | 195,5 g | 224,6 g | 30 g d'acrylate d'éthyl-2-hexyle | 40 nm | 0,18 |
| exemple 8 | 237,4 g | 272,8 g | 15 g de méthacrylate de méthyle | 32 nm | 0,18 |
| exemple 9 | 195,5 g | 224,6 g | 18 g de méthacrylate de méthyle + 12 g d'acrylate de butyle | 41 nm | 0,35 |
| exemple 10 | 195,5 g | 224,6 g | 15 g de méthacrylate de méthyle + 15 g d'acrylate de butyle | 33 nm | 0,1 |

On constate donc que pour tous ces exemples, on obtient une population de particules unique et homogène, dont la taille a été peu modifiée par la polymérisation.

### Exemple 11

a) On compare les propriétés filmogènes des dispersions des polymères selon l'invention, à température ambiante.
   On obtient les résultats suivants:
   . les dispersions des exemples 1, 4, 6, 8, 9 et 10 permettent l'obtention de films homogènes et transparents après séchage,
   . les dispersions des exemples 3, 5 et 7 donnent des films "faïencés" c'est-à-dire présentant des micro-craquelures.
b) On mesure la viscosité, la dureté, la brillance, la polarité et l'énergie de surface de deux dispersions selon l'invention, contenant ou non un épaississant et un colorant.

On obtient les résultats suivants:
. pour les dispersions non colorées, non épaissies

| | viscosité | brillance (noir 60°) | polarité | énergie de surface |
|---|---|---|---|---|
| exemple 1 | liquide | 89,6 | 25,7% | 42,8 mN/m |
| exemple 9 | liquide | 89,9 | 19,8% | 35,8 mN/m |
| SANCUR 815 | | | 33% | 46,7 mN/m |

Les films obtenus avec les dispersions aqueuses des exemples 1 et 9, à 28% de matière sèche, sont très brillants, de polarité et d'énergie de surface légèrement inférieures, mais comparables, à celle de la dispersion de polyuréthanne initiale. On constate également que leur résistance à l'eau est bonne: pas de décollement après 4 heures à 45°C dans une solution aqueuse à 1% de tensioactif (teepol).
. pour les dispersions non colorées, épaissies
On constate que les films obtenus avec une dispersion aqueuse à 28% de matière sèche contenant 0,3% d'épaississant associatif polyuréthanne SER AD FX 1100 (Servo) sont très brillants, de dureté élevées (117,5 pour la dispersion de l'exemple 1 et 89,1 pour celle de l'exemple 9) et que leur résistance à l'eau est bonne.
. pour les dispersions aqueuses selon l'invention (28% de matière sèche), colorées (1,5% de pigments) et épaissies avec SER AD FX 1100 (Servo)

| | épaississant | viscosité (mPa.s) | brillance (noir 60°) | dureté | polarité | énergie de surface |
|---|---|---|---|---|---|---|
| exemple 1 | 0,3% | 110/100 | 81,8 | 88,3 | 32,8% | 47,6 mN/m |
| | 0,56% | 1650/1330 | 83,1 | 167,9 | 33,6% | 49,1 mN/m |
| exemple 9 | 0,3% | 340/250 | 81,9 | 65,8 | 46,2% | 43,7 mN/m |
| | 0,41% | 1100/1020 | 83,3 | 116,7 | 44,9% | 45,9 mN/m |

On constate donc que les films obtenus avec les dispersions selon l'invention, colorées et épaissies, présentent une brillance satisfaisante, des valeurs d'énergies de surface et de polarité classiques ainsi qu'une dureté convenable.

Leur résistance à l'eau est très bonne: pas de décollement après 4 heures à 45°C dans une solution aqueuse à 1% de tensioactif (teepol).

### Exemple 12

On compare les propriétés des films obtenus avec:
. deux dispersions aqueuses selon l'invention (exemples 1 et 9) ayant un taux de matière sèche de 40%
. une dispersion aqueuse de SANCUR 815 à 35,8% de matière sèche
. un mélange de 70% d'une dispersion aqueuse de polyuréthanne SANCUR 815 (taux de matière sèche 35,8%) et de 30% d'un copolymère acrylique méthacrylate de méthyle-acrylate de butyle (60:40), ledit mélange étant à 55% de matière sèche.
Ces films contiennent un épaississant associatif SER AD FX 1100 (0,3% en poids).
On obtient les résultats suivants:

| | épaississant | viscosité (mPa.s) | brillance (noir 60°) | dureté | polarité | énergie de surface (mN/m) |
|---|---|---|---|---|---|---|
| exemple 1 | 0,56% | 1650/1330 | 83,1 | 167,9 | 33,6% | 49,1 |
| exemple 9 | 0,41% | 1100/1020 | 83,3 | 116,7 | 44,9% | 45,9 |
| SANCUR 815 | 0,45% | 530/540 | 87,0 | 178,8 | 39,2% | 52,6 |
| mélange | 0,65% | 1460/1400 | 82,2 | 89,6 | 45,1% | 47,4 |

On constate que les dispersions selon l'invention présentent des propriétés comparables, sur le plan de la brillance et de la dureté, à celles de la dispersion de polyuréthanne seule.
Toutefois la résistance à l'eau des dispersions selon l'invention est nettement supérieure à celle de la dispersion de polyuréthanne seule:
pas de décollement après 4 heures à 45°C dans une solution aqueuse à 1% de tensioactif (teepol), alors que pour la dispersion de SANCUR, on constate au bout de 2 heures, un décollement du film.
Par ailleurs, on constate également que les dispersions selon l'invention sont nettement supérieures, au point de vue dureté, à la dispersion obtenue par simple mélange du polyuréthanne et de l'acrylique.

### Exemple 13

On introduit dans un réacteur, 251,6 g de dispersion aqueuse de polyuréthanne (taux de matière sèche: 31,8 %) vendue sous le nom de SANCUR 11600 par Sancor. On ajoute de l'eau permutée de manière à obtenir une dispersion à 16,6% en poids de matière sèche.

On ajoute, sous azote, 20 g de méthacrylate de cyclohexyle au goutte à goutte, puis on laisse sous agitation pendant 1 heure à 80°C.
On ajoute 0,8 ml de tertbutylperoxy-2-éthylhexanoate (Trigonox 21S de Akzo) et on laisse réagir 8 heures sous agitation et barbotage d'azote à 80°C.
On descend la température du mélange réactionnel à 25°C et l'on concentre la dispersion sous pression réduite jusqu'à obtention d'un taux de matière sèche de 35%.
On obtient ainsi une dispersion qui, après filtration sur toile de Nylon, a les caractéristiques suivantes:
. taille moyenne des particules déterminée par un appareil de diffusion quasi-élastique de lumière du type Coulter N4, de Coultronix: 28 nm
. polydispersité: 0,15
Sachant que la taille des particules dans la dispersion initiale de polyuréthanne (SANCUR 11600) est de 21 nm avec une polydispersité de 0,15, on constate que la polymérisation du monomère n'a presque pas modifié la taille des particules initiales.
La dispersion obtenue est une dispersion aqueuse d'un polymère hybride dont les particules résultent de la polymérisation radicalaire d'un monomère méthacrylate de cyclohexyle , à l'intérieur et/ou partiellement en surface, des particules d'un polymère préexistant de polyuréthanne.

### Exemple 14

On introduit dans un réacteur 74,93 g de dispersion aqueuse de polyuréthanne (taux de matière sèche : 53,4 %) vendue sous le nom de SANCUR 2255 par Sancor. On ajoute de l'eau permutée de manière à obtenir une dispersion aqueuse à 16,6% en poids de matière sèche.
On ajoute 10 g de méthacrylate de méthyle au goutte à goutte, puis on laisse sous agitation pendant 1 heure à 80°C.
On ajoute 0,4 ml de tertbutylperoxy-2-éthylhexanoate (Trigonox 21S de Akzo) et on laisse réagir 8 heures sous agitation et barbotage d'azote à 80°C.
On descend la température du mélange réactionnel à 25°C et l'on concentre la dispersion sous pression réduite jusqu'à obtention d'un taux de matière sèche de 30%.
On obtient ainsi une dispersion qui, après filtration sur toile de Nylon, a les caractéristiques suivantes:
. taille moyenne des particules déterminée par un appareil de diffusion quasi-élastique de lumière du type Coulter N4, de Coultronix: 87 nm
. polydispersité: <0,1
Sachant que la taille des particules dans la dispersion initiale de polyuréthanne (SANCUR 2255) est de 87 nm avec une polydispersité inférieure à 0,1, on constate que la polymérisation du monomère n'a presque pas modifié la taille des particules initiales.

La dispersion obtenue est une dispersion aqueuse d'un polymère hybride dont les particules résultent de la polymérisation radicalaire d'un monomère méthacrylate de méthyle, à l'intérieur et/ou partiellement en surface, des particules d'un polymère préexistant de polyuréthanne.

### Exemple 15

On introduit dans un réacteur, 251,6 g de dispersion aqueuse de polyuréthanne (taux de matière sèche : 31,8 %) vendue sous le nom de SANCUR 11600 par Sancor. On ajoute de l'eau permutée de manière à obtenir une dispersion à 16,6% en poids de matière sèche.
On ajoute, sous azote, 20 g de méthacrylate de méthyle au goutte à goutte, puis on laisse sous agitation pendant 1 heure à 80°C.
On ajoute 0,8 ml de tertbutylperoxy-2-éthylhexanoate (Trigonox 21S de Akzo) et on laisse réagir 8 heures sous agitation et barbotage d'azote à 80°C.
On descend la température du mélange réactionnel à 25°C et l'on concentre la dispersion sous pression réduite jusqu'à obtention d'un taux de matière sèche de 35%.
On obtient ainsi une dispersion qui, après filtration sur toile de Nylon, a les caractéristiques suivantes:
. taille moyenne des particules déterminée par un appareil de diffusion quasi-élastique de lumière du type Coulter N4, de Coultronix: 30 nm
. polydispersité: 0,20
Sachant que la taille des particules dans la dispersion initiale de polyuréthanne (SANCUR 11600) est de 21 nm avec une polydispersité de 0,15, on constate que la polymérisation du monomère n'a presque pas modifié la taille des particules initiales.
La dispersion obtenue est une dispersion aqueuse d'un polymère hybride dont les particules résultent de la polymérisation radicalaire d'un monomère méthacrylate de méthyle, à l'intérieur et/ou partiellement en surface, des particules d'un polymère préexistant de polyuréthanne.

### Exemple 16

On prépare un vernis à ongles ayant la composition suivante:

| | |
|---|---|
| . dispersion de l'exemple 6 (matière sèche 40%) | 70% |
| . épaississant polyuréthanne associatif SER AD FX 1100 (Servo) | 0,47% |
| . tensioactif siliconé KF 355A (Shin Etsu) | 0,5% |
| . pigments | 1,0% |
| . propylène glycol | 0,3% |
| . eau | qsp 100% |

On obtient ainsi un vernis à ongles qui s'étale facilement, présentant une dureté, une brillance et une résistance à l'eau adéquates.

### Exemple 17

On prépare un vernis à ongles ayant la composition suivante:

| | |
|---|---|
| . dispersion de l'exemple 10 (matière sèche 40%) | 70% |
| . épaississant polyuréthanne associatif SER AD FX 1100 (Servo) | 0,3% |
| . tensioactif siliconé KF 355A (Shin Etsu) | 0,5% |
| . pigments | 1,0% |
| . propylène glycol | 0,3% |
| . eau | qsp 100% |

On obtient ainsi un vernis à ongles qui s'étale facilement, présentant une dureté, une brillance et une résistance à l'eau adéquates.

### Exemple 18

On prépare un vernis à ongles à partir de la dispersion obtenue à l'exemple 13. On dilue la dispersion obtenue à l'exemple 13 de manière à obtenir un taux de matière sèche de 30% en poids.
On ajoute 6,4 g de lactate d'éthyle et on laisse agiter pendant 24 heures à température ambiante. On concentre alors la dispersion jusqu'à un taux de matière séche de 40% en poids.
Le lactate d'éthyle permet de favoriser la filmification des particules de polymère contenu dans la dispersion, en abaissant sa température de transition vitreuse; le lactate d'éthyle s'évapore après l'application de la composition et l'on obtient un film d'une rigidité convenable.
La composition de vernis à ongles comprend:

| | |
|---|---|
| . dispersion ci-dessus (matière sèche 40%) | 70% |
| . épaississant polyuréthanne associatif SER AD FX 1100 (Servo) | 0,47% |
| . tensioactif siliconé KF 355A (Shin Etsu) | 0,5% |
| . pigments | 1,0% |
| . propylène glycol | 0,3% |
| . eau | qsp 100% |

On obtient ainsi un vernis à ongles qui s'étale facilement, présentant une dureté, une brillance et une résistance à l'eau adéquates.

### Exemple 19

La dispersion comprenant le lactate d'éthyle préparée à l'exemple 18 est diluée jusqu'à un taux de matière sèche de 5% en poids.
Cette dispersion peut être employée en tant que lotion de Brushing et permet une bonne fixation de la coiffure, ladite fixation pouvant persister à deux shampooings ultérieurs.

### Exemple 20

On prépare une lotion de Brushing à partir de la dispersion obtenue à l'exemple 14.

On dilue la dispersion obtenue à l'exemple 14 de manière à obtenir un taux de matière sèche de 30% en poids.
On ajoute 5,35 g d'agent de coalescence vendu sous le nom DOWANOL PMA par Dow Corning, et on laisse agiter pendant 24 heures à température ambiante. Le DOWANOL permet de favoriser la filmification des particules de polymère contenu dans la dispersion, en abaissant sa température de transition vitreuse; il s'évapore après l'application de la composition.

On dilue la dispersion jusqu'à un taux de matière sèche de 5% en poids.
Cette dispersion peut être employée en tant que lotion de Brushing et permet une bonne fixation de la coiffure.

### Exemple 21

On dilue la dispersion obtenue à l'exemple 15 jusqu'à un taux de matière sèche de 17% en poids.
On prépare une composition de mascara de la manière suivante.
On mélange 11,5 g de stéarate de triéthanolamine, 7,0 g de cires d'abeilles, 4,1 g de cire de Carnauba et 11,4 g de paraffine. On porte le mélange à 85°C et on y ajoute 5,5 g d'oxyde de fer noir.
On prépare un second mélange comprenant 35 ml d'eau, 4,5 g de gomme arabique et 0,16 g d'hydroxyéthylcellulose, que l'on fait chauffer à 85°C.
On combine les deux mélanges et l'on ajoute 21,3 g de la dispersion à 17% de matière sèche.
On obtient un mascara présentant de bonnes caractéristiques cosmétiques à l'application sur les cils et une bonne rémanence à l'eau.

## Revendications

1. Utilisation d'une dispersion aqueuse de polymère constituée par des particules résultant de la polymérisation radicalaire d'au moins un monomère radicalaire, à l'intérieur et/ou partiellement en surface, de particules préexistantes d'au moins un polymère choisi dans le groupe constitué par les polyuréthannes et/ou les polyurées, comme agent filmogène dans une composition cosmétique ou pharmaceutique.

2. Utilisation selon la revendication 1, dans laquelle le polymère choisi parmi les polyuréthannes et/ou les polyurées est un copolymère polyuréthanne, polyurée/uréthanne ou polyurée, aliphatique, cycloaliphatique ou aromatique, comportant, seule ou en mélange,
. au moins une séquence d'origine polyester aliphatique linéaire ou ramifié et/ou cycloaliphatique et/ou aromatique, et/ou
. au moins une séquence d'origine polyéther aliphatique et/ou cycloaliphatique et/ou aromatique, et/ou
. au moins une séquence siliconée, substituée ou non, ramifiée ou non, par exemple polydiméthylsiloxane ou polyméthylphénylsiloxane, et/ou
. au moins une séquence comportant des groupes fluorés.

3. Utilisation selon l'une des revendications 1 à 2, dans laquelle le monomère est choisi dans le groupe constitué par les esters d'acide acrylique ou méthacrylique; les acrylamides ou méthacrylamides N-substituées ou N,N-substituées; les esters vinyliques; le styrène; les monomères vinylique, acrylique ou méthacrylique comportant un ou plusieurs groupes siloxanes; les monomères vinylique, allylique, ester éther ou amide de l'acide acrylique ou métacrylique comportant un ou plusieurs groupes halogénés, en particulier chlorés et/ou fluorés, et/ou comportant un groupe absorbant dans l'UVA et/ou l'UVB , en particulier les groupements benzylidène camphre et benzotriazole, substitués ou non.

4. Utilisation selon la revendication 3, dans laquelle le groupe siloxane est choisi parmi :
. le monomère de formule
CH₂=C(CH₃)-C(O)-O-(CH₂)₃-Si-[O-Si(CH₃)₃]₃
. un macromonomère siliconé à terminaison monofonctionnelle vinylique, allylique, ester éther ou amide de l'acide acrylique ou méthacrylique, de formule
CH₂=C(R1)-C(O)-X-(CH₂)ₚ-[Si(CH₃)(R4)-O-]ₙ-Si(CH₃)₂-R3
dans laquelle R1 représente H ou CH₃, X représente O ou NH, p est un entier pouvant être nul, R3 et R4 représentent de manière indépendante CH₃ ou un groupe aliphatique, cycloaliphatique ou aromatique, et n est un entier.

5. Utilisation selon l'une des revendications 1 à 4, dans un produit destiné à la photoprotection de la peau et/ou des cheveux contre le rayonnement ultraviolet, en particulier contre le rayonnement solaire; dans un produit capillaire tel qu'une laque aérosol, un shampooing, une lotion de coiffage, une mousse de coiffage; dans un produit de maquillage tel qu'un vernis à ongles ou un mascara; dans une base de soin pour ongles ou un produit de soin pour la peau.

6. Composition cosmétique ou pharmaceutique comprenant une dispersion aqueuse de polymère constituée par des particules résultant de la polymérisation radicalaire d'au moins un monomère radicalaire, à l'intérieur et/ou partiellement en surface, de particules préexistantes d'une dispersion aqueuse de polyuréthanne anionique, cationique ou amphotère, de polyester-polyuréthanne, de polyéther-polyuréthanne et/ou de polyurée, seul ou en mélange.

7. Composition selon la revendication 6, dans laquelle le polymère choisi parmi les polyuréthannes et les polyurées est un copolymère polyuréthanne, polyurée/uréthanne ou polyurée, aliphatique, cycloaliphatique ou aromatique, comportant, seule ou en mélange,
. au moins une séquence d'origine polyester aliphatique linéaire ou ramifié et/ou cycloaliphatique et/ou aromatique, et/ou
. au moins une séquence d'origine polyéther aliphatique et/ou cycloaliphatique et/ou aromatique, et/ou
. au moins une séquence siliconée, substituée ou non, ramifiée ou non, par exemple polydiméthylsiloxane ou polyméthylphénylsiloxane, et/ou
. au moins une séquence comportant des groupes fluorés.

8. Composition selon l'une des revendications 6 - 7, dans laquelle le monomère est choisi dans le groupe constitué par les esters d'acide acrylique ou méthacrylique; les acrylamides ou méthacrylamides N-substituées ou N,N-substituées; les esters vinyliques; le styrène; les monomères vinylique, acrylique ou méthacrylique comportant un ou plusieurs groupes siloxanes; les monomères vinylique, allylique, ester éther ou amide de l'acide acrylique ou métacrylique comportant un ou plusieurs groupes halogénés, en particulier chlorés et/ou fluorés, et/ou comportant un groupe absorbant dans l'UVA et/ou l'UVB , en particulier les groupements benzylidène camphre et benzotriazole, substitués ou non.

9. Composition selon la revendication 8, dans laquelle le groupe siloxane est choisi parmi :
. le monomère de formule
CH₂=C(CH₃)-C(O)-O-(CH₂)₃-Si-[O-Si(CH₃)₃]₃
. un macromonomère siliconé à terminaison monofonctionnelle vinylique, allylique, ester éther ou amide de l'acide acrylique ou méthacrylique, de formule
CH₂=C(R1)-C(O)-X-(CH₂)ₚ-[Si(CH₃)(R4)-O-]ₙ-Si(CH₃)₂-R3
dans laquelle R1 représente H ou CH₃, X représente O ou NH, p est un entier pouvant être nul, R3 et R4 représentent de manière indépendante CH₃ ou un groupe aliphatique, cycloaliphatique ou aromatique, et n est un entier.

10. Composition selon l'une des revendications 6 - 9, se présentant sous forme d'un produit capillaire tel qu'une laque aérosol, un shampooing, une lotion de coiffage, une mousse de coiffage; d'un produit de maquillage tel qu'un mascara ou un vernis à ongles; d'une base de soin pour les ongles ou d'un produit destiné à la photoprotection de la peau et/ou des cheveux contre le rayonnement ultraviolet, en particulier contre le rayonnement solaire.

## Claims

1. Use of a polymer aqueous dispersion consisting of particles resulting from the radical polymerization of at least one radical monomer, within and/or partially at the surface, of preexisting particles of at least one polymer chosen from the group consisting of polyurethanes and/or polyureas, as film-forming agent in a cosmetic or pharmaceutical composition.

2. Use according to Claim 1, in which the polymer chosen from polyurethanes and/or polyureas is an aliphatic, cycloaliphatic or aromatic polyurethane, polyurea/urethane or polyurea copolymer, containing, alone or as a mixture,
. at least one sequence of linear or branched aliphatic and/or cycloaliphatic and/or aromatic polyester origin, and/or
. at least one sequence of aliphatic and/or cycloaliphatic and/or aromatic polyether origin, and/or
. at least one branched or unbranched, substituted or unsubstituted silicone-containing sequence, for example polydimethylsiloxane or polymethylphenylsiloxane, and/or
. at least one sequence containing fluorinated groups.

3. Use according to either of Claims 1 and 2, in which the monomer is chosen from the group consisting of acrylic or methacrylic acid esters; N-substituted or N,N-substituted acrylamides or methacrylamides; vinyl esters; styrene; vinyl, acrylic or methacrylic monomers containing one or more siloxane groups; vinyl, allyl, ester ether or acrylamide or methacrylamide monomers containing one or more halogenated groups, in particular chlorinated and/or fluorinated groups, and/or containing a group absorbing in the UVA and/or UVB region, in particular benzylidenecamphor and benzotriazole groups, which may or may not be substituted.

4. Use according to Claim 3, in which the siloxane group is chosen from:
. the monomer of formula
CH₂=C(CH₃)-C(O)-O-(CH₂)₃-Si-[O-Si(CH₃)₃]₃
. a silicone-containing macromonomer containing a vinyl, allyl, ester ether or acrylamide or methacrylamide monofunctional end group, of formula
CH₂=C(R1)-C(O)-X-(CH₂)ₚ-[Si(CH₃)(R4)-O-]ₙ-Si(CH₃)₂-R3
in which R1 represents H or CH₃, X represents O or NH, p is an integer which may be zero, R3 and R4 independently represent CH₃ or an aliphatic, cycloaliphatic or aromatic group, and n is an integer.

5. Use according to one of Claims 1 to 4, in a product intended for the photoprotection of the skin and/or the hair against ultraviolet radiation, in particular against solar radiation; in a hair product such as an aerosol fixing hairspray, a shampoo, a styling lotion or a styling mousse; in a make-up product such as a nail varnish or a mascara; in a care base for the nails or a skin care product.

6. Cosmetic or pharmaceutical composition comprising an aqueous dispersion of polymer consisting of particles resulting from the radical polymerization of at least one radical monomer, within and/or partially at the surface, of preexisting particles of an aqueous dispersion of anionic, cationic or amphoteric polyurethane, of polyester-polyurethane, of polyether-polyurethane and/or of polyurea, alone or as a mixture.

7. Composition according to Claim 6, in which the polymer chosen from polyurethanes and polyureas is an aliphatic, cycloaliphatic or aromatic polyurethane, polyurea/urethane or polyurea copolymer, containing, alone or as a mixture,
. at least one sequence of linear or branched aliphatic and/or cycloaliphatic and/or aromatic polyester origin, and/or
. at least one sequence of aliphatic and/or cycloaliphatic and/or aromatic polyether origin, and/or
. at least one branched or unbranched, substituted or unsubstituted silicone-containing sequence, for example polydimethylsiloxane or polymethylphenylsiloxane, and/or
. at least one sequence containing fluorinated groups.

8. Composition according to either of Claims 6 and 7, in which the monomer is chosen from the group consisting of acrylic or methacrylic acid esters; N-substituted or N,N-substituted acrylamides or methacrylamides; vinyl esters; styrene; vinyl, acrylic or methacrylic monomers containing one or more siloxane groups; vinyl, allyl, ester ether or acrylamide or methacrylamide monomers containing one or more halogenated groups, in particular chlorinated and/or fluorinated groups, and/or containing a group absorbing in the UVA and/or UVB region, in particular benzylidenecamphor and benzotriazole groups, which may or may not be substituted.

9. Composition according to Claim 8, in which the siloxane group is chosen from:
. the monomer of formula
CH₂=C(CH₃)-C(O)-O-(CH₂)₃-Si-[O-Si(CH₃)₃]₃
. a silicone-containing macromonomer containing a vinyl, allyl, ester ether or acrylamide or methacrylamide monofunctional end group, of formula
CH₂=C(R1)-C(O)-X-(CH₂)ₚ-[Si(CH₃)(R4)-O-]ₙ-Si(CH₃)₂-R3
in which R1 represents H or CH₃, X represents O or NH, p is an integer which may be zero, R3 and R4 independently represent CH₃ or an aliphatic, cycloaliphatic or aromatic group, and n is an integer.

10. Composition according to one of Claims 6 - 9, which is in the form of a hair product such as an aerosol fixing hairspray, a shampoo, a styling lotion or a styling mousse; a make-up product such as a mascara or a nail varnish; a care base for the nails or a product intended for the photoprotection of the skin and/or the hair against ultraviolet radiation, in particular against solar radiation.

## Patentansprüche

1. Verwendung einer wäßrigen Polymerdispersion, die aus Partikeln besteht, die bei der radikalischen Polymerisation mindestens eines radikalischen Monomers im Innern und/oder teilweise an der Oberfläche von bereits vorhandenen Partikeln aus mindestens einem Polymer entstehen, das unter Polyurethanen und/oder Polyharnstoffen ausgewählt ist, als filmbildendes Mittel in kosmetischen oder pharmazeutischen Zusammensetzungen.

2. Verwendung nach Anspruch 1, worin das unter Polyurethanen und/oder Polyharnstoffen ausgewählte Polymer ein aliphatisches, cycloaliphatisches oder aromatisches Polyurethan-, Polyharnstoff/Urethan- oder Polyharnstoff-Copolymer ist, das, allein oder im Gemisch, folgende Gruppen enthält:
. mindestens eine Sequenz, die von einem geradkettigen oder verzweigten aliphatischen und/oder cycloaliphatischen und/oder aromatischen Polyester stammt, und/oder
. mindestens eine Sequenz, die von einem aliphatischen und/oder cycloaliphatischen und/oder aromatischen Polyether stammt, und/oder
. mindestens eine ggf. verzweigte, ggf. substituierte Siliconsequenz, beispielsweise Polydimethylsiloxan oder Polymethyl-phenylsiloxan, und/oder
. mindestens eine Sequenz, die fluorierte Gruppen enthält.

3. Verwendung nach einem der Ansprüche 1 bis 2, worin das Monomer ausgewählt ist unter Acrylsäureestern und Methacrylsäureestern; N-substituierten oder N,N-substituierten Acrylamiden und Methacrylamiden; Vinylestern; Styrol; Vinyl-, Acryl- und Methacrylmonomeren, die eine oder mehrere Siloxangruppen enthalten; Vinylmonomeren, Allylmonomeren, Estern und Amiden von Acrylsäure oder Methacrylsäure, die eine oder mehrere halogenhaltige, insbesondere chlor- und/oder fluorhaltige, Gruppen enthalten und/oder eine im UV-A- und/oder UV-B-Bereich absorbierende Gruppe aufweisen, insbesondere die ggf. substituierte Benzylidencamphergruppe oder die ggf. substituierte Benzotriazolgruppe.

4. Verwendung nach Anspruch 3, wobei die Siloxangruppe ausgewählt ist unter
. dem Monomer der Formel
CH₂=C(CH₃)-C(O)-O-(CH₂)₃-Si-[O-Si(CH₃)₃]₃
und
. endständig monofunktionellen siliconhaltigen Makromonomeren mit einer Vinyl- oder Allyl-Endgruppe oder einer Endgruppe eines Esters oder Amids der Acrylsäure oder Methacrylsäure der Formel
CH₂=C(R1)-C(O)-X-(CH₂)ₚ-[Si(CH₃)(R4)-O-]ₙ-Si(CH₃)₂-R3,
worin bedeuten:
. R1 H oder CH₃,
. X Sauerstoff oder -NH-,
. p eine ganze Zahl oder Null sein kann,
. R3 und R4 unabhängig voneinander -CH₃ oder eine aliphatische, cycloaliphatische oder aromatische Gruppe und
. n eine ganze Zahl.

5. Verwendung nach einem der Ansprüche 1 bis 5 in einem Produkt, das zum Lichtschutz der Haut und/oder der Haare gegen UV-Strahlung, insbesondere gegen Sonnenstrahlung, bestimmt ist, in einem Produkt zur Haarbehandlung, wie beispielsweise einem Aerosol-Haarlack, einem Haarwaschmittel, einer Frisierlotion und einem Frisierschaum, in einem Produkt zum Schminken, wie einem Nagellack oder einer Mascara und in einer Basisformulierung zur Pflege der Nägel oder einem Pflegeprodukt für die Haut.

6. Kosmetische oder pharmazeutische Zusammensetzung, die eine wäßrige Polymerdispersion enthält, die aus Partikeln besteht, die bei der radikalischen Polymerisation mindestens eines radikalisch polymerisierbaren Monomers im Inneren und/oder teilweise an der Oberfläche von bereits vorhandenen Partikeln einer wäßrigen Dispersion eines anionischen, kationischen oder amphoteren Polyurethans, eines Polyester-Polyurethans, eines Polyether-Polyurethans und/oder eines Polyharnstoffs, allein oder im Gemisch, entstehen.

7. Zusammensetzung nach Anspruch 6, wobei das unter Polyurethanen und Polyharnstoffen ausgewählte Polymer ein aliphatisches, cycloaliphatisches oder aromatisches Polyurethan-, Polyharnstoff/Urethan- oder Polyharnstoff-Copolymer ist, das, allein oder im Gemisch, enthält:
. mindestens eine Sequenz, die von einem linearen oder verzweigten aliphatischen und/oder cycloaliphatischen und/oder aromatischen Polyester stammt, und/oder
. mindestens eine Sequenz, die von einem aliphatischen und/oder cycloaliphatischen und/oder aromatischen Polyether stammt, und/oder
. mindestens eine ggf. verzweigte, ggf. substituierte Siliconsequenz, beispielsweise Polydimethyl-siloxan oder Polymethylphenylsiloxan, und/oder
. mindestens eine Gruppe, die fluorierte Gruppen aufweist.

8. Zusammensetzung nach einem der Ansprüche 6 bis 7, worin das Monomer ausgewählt ist unter Acrylsäureestern und Methacrylsäureestern; N-substituierten und N,N-substituierten Acrylamiden und Methacrylamiden, Vinylestern, Styrol, Vinyl-, Acryl- und Methacrylmonomeren, die eine oder mehrere Siloxangruppen enthalten; Vinylmonomeren, Allylmonomeren, Estern und Amiden von Acrylsäure oder Methacrylsäure, die eine oder mehrere halogenhaltige Gruppen, insbesondere chlorhaltige und/oder fluorhaltige, Gruppen enthalten und/oder einen UV-A- und/oder UV-B-Bereich absorbierende Gruppe aufweisen, insbesondere die ggf. substituierte Benzylidencamphergruppe und die ggf. substituierte Benzotriazolgruppe.

9. Zusammensetzung nach Anspruch 8, wobei die Siloxangruppe ausgewählt ist unter
. dem Monomer der Formel
CH₂=C(CH₃)-C(O)-O-(CH₂)₃-Si-[O-Si(CH₃)₃]₃
. endstädnig monofunktionellen siliconhaltigen Makromonomeren mit einer Vinyl- oder Allyl-Endgruppe oder einer Endgruppe eines Esters oder Amids der Acrylsäure oder Methacrylsäure der Formel
CH₂=C(R1)-C(O)-X-(CH₂)ₚ-[Si(CH₃)(R4)-O-]ₙ-Si(CH₃)₂-R3,
worin bedeuten:
. R1 Wasserstoff oder -CH₃,
. X Sauerstoff oder -NH-,
. p eine ganze Zahl oder Null sein kann,
. R3 und R4 unabhängig voneinander -CH₃ oder eine aliphatische, cycloaliphatische oder aromatische Gruppe, und
. n eine ganze Zahl.

10. Zusammensetzung nach einem der Ansprüche 6 bis 9, die als Produkt zur Haarbehandlung, wie z.B. als Aerosol-Haarlack, Haarwaschmittel, Frisierlotion und Frisierschaum, als Produkt zum Schminken, z.B. als Mascara oder Nagellack, als Pflegeprodukt für die Nägel oder als Produkt, das zum Lichtschutz der Haut und/oder der Haare gegen UV-Strahlung und insbesondere gegen Sonnenlicht bestimmt ist, vorliegt.
